# FASCICULE DE BREVET EUROPEEN

(11) **EP 0 652 865 B1**
(45) Date de publication et mention de la délivrance du brevet: **12.03.1997**
(21) Numéro de dépôt: 94917696.0
(22) Date de dépôt: 01.06.1994
(51) Int. Cl.: C07C 323/14, C07C 317/18, A61K 7/00

(54) **COMPOSES HYDRO-FLUORO-CARBONES ET LEUR UTILISATION DANS DES COMPOSITIONS COSMETIQUES**
HYDROFLUORKOHLENSTOFF-VERBINDUNGEN UND IHRE VERWENDUNG IN KOSMETISCHEN ZUBEREITUNGEN
HYDROFLUOROCARBON-CONTAINING COMPOUNDS AND THEIR USE IN COSMETIC COMPOSITIONS

(30) Priorité: 02.06.1993 FR 9306606
(43) Date de publication de la demande: 17.05.1995
(73) Titulaire: L'OREAL, 75008 Paris (FR)
(72) Inventeur: BOLLENS, Eric, F-94410 Saint-Maurice (FR); MAHIEU, Claude, F-75017 Paris (FR)
(74) Mandataire: Casalonga, Axel
(86) Numéro de dépôt international: FR9400635
(87) Numéro de publication internationale: WO9427960

(56) Documents cités:
- EP-A- 0 010 523
- WO-A-93/11103
- FR-A- 2 416 222
- FR-A- 2 516 920
- US-A- 4 126 702

## Description

La présente invention concerne des composés hydro- et fluorocarbonés utiles notamment dans des compositions cosmétiques, ainsi que des compositions cosmétiques comportant ces composés.

Il est connu d'utiliser des perfluoropolyéthers, notamment dans des procédés de nettoyage, de protection, de maquillage de la peau ou bien de lavage des cheveux. Ces composés sont connus pour leur basse tension superficielle et leur facilité d'étalement, mais présentent une solubilité dans la plupart des fluides très réduite, excepté dans les fluides fluorés, ce qui rend très difficile leur formulation dans des compositions cosmétiques. Certains de ces composés, les perfluorométhylisopropyléthers, sont connus sous le nom de "FOMBLIN HC", commercialisés par la Société MONTEFLUO.

La demanderesse a maintenant découvert des composés nouveaux qui, contrairement aux FOMBLIN connus, présentent une bonne solubilité, notamment dans les solvants classiques utilisés en cosmétique comme les alcools inférieurs, ainsi que les corps gras et huiles usuels. Leur caractère amphiphile, leurs propriétés et leur compatibilité avec les solvants permettent notamment de préparer des compositions homogènes et stables, et par exemple d'assurer une bonne stabilité des émulsions dans lesquelles ils interviennent.

Ainsi, la présente invention concerne les composés de formule :

R_{F} - (CH₂)ₙ - X - [C₃H₅(OH)] - (Y)ₓ - R_{H} (I)

dans laquelle
C₃H₅(OH) représente les structures : 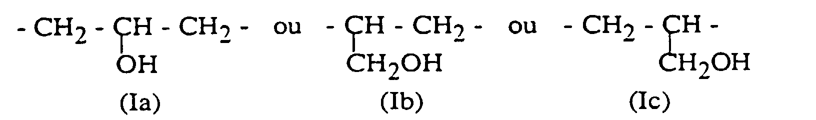
R_{F} représente un radical alkyle perfluoré, linéaire ou ramifié en C₄-C₂₀ ou un mélange de radicaux alkyle perfluorés, linéaires ou ramifiés en C₄-C₂₀;
R_{H} représente un radical alkyle linéaire ou ramifié en C₂₃-C₃₆ ou un mélange de radicaux alkyle linéaires ou ramifiés en C₂₃-C₃₆;
n est compris entre 0 et 4
X représente O, S, 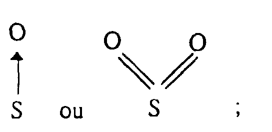
x représente O ou 1 ;
Y représente O, S,

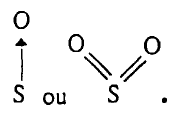

Parmi ces composés, les composés préférés sont ceux pour lesquels R_{F} désigne un radical alkyle perfluoré en C₆-C₁₀, R_{H} désigne un radical alkyle linéaire ou ramifié en C₂₃-C₃₀, n est 2, et x est 1.

De préférence, X est S et Y est O.

Parmi les radicaux alkyle linéaires ou ramifiés, on peut citer notamment les radicaux 2-décyl-tétradécyle, 2-dodécyl-hexadécyle, 2-hexadécyl-eicosyle.

WO-A-9311103 décrit des composés hydro-fluoro-carbonés de formule (I) mais où le reste R_{H} représente un radical alkyle en C₁-C₂₂ et leur utilisation dans des compositions cosmétiques. Ce document appartient à l'état de la technique tel que défini à l'Article 54(3)(4) CBE.

Les composés de formule (I) selon l'invention peuvent et préparés en mettant en oeuvre la réaction d'un composé fluoré hydrogène acide de formule (II):

R_{F}-(CH₂)ₙ-X-H (II)

avec un époxyde de formule (III) : 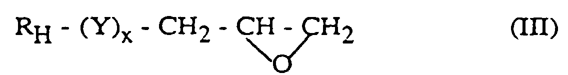 ou la réaction d'un composé hydrocarboné à hydrogène acide de formule (IV) :

R_{H} - (Y)_{X} - H (IV)

avec un époxyde fluoré de formule (V) : 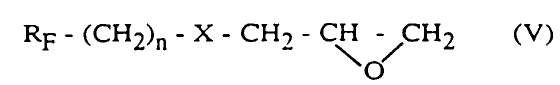 en présence d'un composé basique ou acide jouant le rôle de réactif ou de catalyseur, pour obtenir le composé de formule (I) correspondant, les substituants R_{F}, R_{H}, n et x ayant dans les formules (II) et (III), (IV) et (V), la même signification que dans la formule (I) et X et Y désignant O ou S,
et en oxydant éventuellement la fonction mercaptan en sulfoxyde ou sulfone avec un oxydant, de préférence avec de l'eau oxygénée en milieu acide.

Les composés de formule (V) sont décrits notamment dans le brevet US 3976698 ou dans les demandes de brevet EP 300358 et DE 2018461.

Lorsque X représente S dans la formule (II) ou (IV), on utilise de préférence un composé basique.

Les composés jouant le rôle de réactif ou de catalyseur peuvent donc être basiques, tels que les métaux alcalins, les hydroxydes de métaux alcalins ou alcalino-terreux, les alcoolates des métaux alcalins tels que les méthylates ou tertiobutylates, les hydrures alcalins tels que l'hydrure de sodium, les amines tertiaires telles que la pyridine ou la triéthylamine. Ils peuvent aussi être des bases de Lewis parmi lesquelles on peut citer les fluorures de césium, de rubidium, de potassium. Ces composés peuvent être également supportés sur un solide tel que l'alumine. De préférence, on utilise un alcoolate alcalin comme le méthylate de sodium ou une amine tertiaire comme la pyridine.

Ces composés peuvent également être acides, notamment lorsque le produit de départ de formule (II) ou (IV) est un alcool. De tels acides peuvent être les acides minéraux ou leurs sels avec des amines tertiaires ou encore des acides dits de Lewis tels que le trifluorure de bore, le tétrachlorure d'étain, le pentachlorure d'antimoine, utilisés seuls, en solution ou associés à un support usuel.

La concentration des composés acides ou basiques jouant le rôle de réactif ou de catalyseur et mis en oeuvre dans la réaction de préparation des composés de formule (I) peut être comprise entre 1 et 100% molaire, de préférence entre 2 et 10% molaire par rapport au composé fluoré à hydrogène acide de formule (II) ou (IV).

La réaction de préparation peut être mise en oeuvre en présence de solvant ou en l'absence de solvant.

Comme solvant, on peut utiliser des hydrocarbures aliphatiques tels que l'heptane, l'hexane ou des hydrocarbures cycliques tels que le cyclohexane, des hydrocarbures aromatiques tels que le toluène, des éthers tels que l'éther éthylique ou isopropylique ou le tert.-butylméthyléther, des éthers cycliques tels que le dioxanne, ou bien encore l'acétonitrile, le diméthylformamide, la N-méthylpyrrolidone, le diméthylacétamide.

Lorsque le composé de formule (II) ou (IV) est un thiol (X=S), les alcools tels que le méthanol, l'éthanol ou l'isopropanol peuvent aussi être utilisés comme solvant.

Pour préparer les composés selon l'invention, on peut mélanger d'abord le composé à hydrogène acide de formule (II) ou (IV) avec le réactif ou catalyseur acide ou basique, sous atmosphère inerte. Pour réaliser le mélange, on peut opérer à une température comprise entre 20 et 180°C, de préférence entre 50 et 150°C. Par atmosphère inerte, on entend par exemple une atmosphère d'azote, d'argon ou d'hélium.

Le mélange peut être effectué, selon la nature du composé à hydrogène acide et du réactif ou catalyseur, en l'absence ou en présence de solvant.

On ajoute au mélange obtenu l'époxyde de formule (III) ou (V), cette addition pouvant s'effectuer en une seule fois ou progressivement, sur une période pouvant aller de 30 minutes à 2 heures par exemple.

Le temps de réaction est alors compris entre environ 1 heure et 24 heures, de préférence entre 1 heure et 3 heures.

Le composé issu de la réaction peut être oxydé lorsque celui-ci contient une fonction mercaptan, en sulfoxyde ou en sulfone, en présence d'eau oxygénée en milieu acide, selon des méthodes connues, notamment dans les demandes de brevet français FR 2 099 092 et FR 2 516 920.

Il peut être en outre nécessaire de neutraliser le mélange obtenu, et on peut séparer le composé synthétisé de façon usuelle, par distillation, par exemple.

Lorsque la réaction de préparation des composés de formule (I) est effectuée en présence de composé basique, elle conduit uniquement à des composés pour lesquels C₃H₅(OH) représente le groupement (Ia). Dans le cas où la réaction est effectuée en présence de composé acide, on peut obtenir un mélange de composés correspondant aux significations (Ia), (Ib) et (Ic) de C₃H₅(OH).

Les composés selon l'invention peuvent se présenter sous forme d'huile ou sous forme solide à la température ambiante.

Un autre objet de l'invention concerne l'utilisation des produits de formule (I), composés amphiphiles, dans des compositions cosmétiques.

De façon générale, ces composés utilisés dans des compositions cosmétiques permettent d'en améliorer les propriétés cosmétiques. Ils confèrent de la douceur, de la brillance et un toucher non collant aux matières kératiniques telles que la peau, les cheveux et les ongles.

C'est pourquoi la présente invention concerne également des compositions cosmétiques caractérisées en ce qu'elles comportent au moins un composé de formule (I) définie ci-dessus.

Ces compositions peuvent se présenter sous forme d'émulsions, de laits ou de crèmes, de lotions huileuses ou oléoalcooliques, sous forme de gels gras ou oléoalcooliques, de dispersions vésiculaires à base de lipides amphiphiles ioniques ou non-ioniques, de bâtonnets solides, de pâte, de spray ou de mousse aérosol.

Selon la forme des compositions auxquelles sont incorporés les composés de l'invention, ces compositions comportent par ailleurs les adjuvants et additifs usuels pour la forme choisie.

Plus précisément, ces compositions peuvent être des laits et des crèmes pour les soins de la peau ou des cheveux, des crèmes, lotions ou laits démaquillants, des crèmes, gels, laits ou lotions anti-solaire, des crèmes ou mousses de rasage, des lotions après-rasage, des shampooings ou des après-shampooings, des déodorants corporels, des pâtes dentifrices, des laques, des gels de coiffage, des compositions de coloration capillaire, des compositions pour la déformation permanente des cheveux, des produits de soin des lèvres ou des ongles.

Ces compositions cosmétiques peuvent être également utilisées comme produit de maquillage des cils, des sourcils, des ongles, des lèvres ou de la peau comme les crèmes de traitement de l'épiderme, des fonds de teint, des bâtons de rouge à lèvres, des fards à paupières ou à joues, des eye-liners ou mascara, des vernis à ongles, par exemple.

Selon l'invention, les composés de formule (I) représentent de 0,1 à 25%, et de préférence de 0,1 à 15% du poids total de la composition.

Parmi les adjuvants cosmétiques usuels pour ce genre de composition, peuvent être présents en outre dans les compositions selon l'invention, des corps gras usuels, des solvants organiques, des silicones, les épaississants, les adoucissants, des filtres solaires UV-A ou UV-B ou à bande large, des agents anti-mousses, des agents hydratants, des humectants, des parfums, des conservateurs, des tensio-actifs, des charges, des séquestrants, des émulsionnants, des polymères anioniques, cationiques, non-ioniques, amphotères ou leurs mélanges, des anti-perspirants, des agents alcalinisants, des colorants, des pigments, des agents propulseurs, des anti-oxydants et des anti-radicaux libres.

Plus précisément, comme corps gras, on peut utiliser une huile ou une cire ou leur mélange, des acides gras, des alcools gras, des esters d'acides gras tels que les triglycérides d'acides gras en C₆ à C₁₈, de la vaseline, de la paraffine, de la lanoline, de la lanoline hydrogénée ou acétylée.

Parmi les huiles, on peut citer les huiles minérales, animales, végétales ou les huiles de synthèse, et notamment l'huile de vaseline, de paraffine, de ricin, de jojoba, de sésame, ainsi que les huiles et des gommes de silicone et les isoparaffines.

Parmi les cires animales, fossiles, végétales, minérales ou de synthèse, on peut notamment citer les cires d'abeille, de Caroube, de Candelila, les ozokérites, les cires microcristallines ainsi que les cires et résines de silicone.

Parmi les solvants organiques usuellement utilisés dans les compositions cosmétiques, on peut citer plus précisément les mono-alcools ou polyalcools inférieurs en C₁ à C₆ comme l'éthanol, l'isopropanol, le propylèneglycol, le glycérol, le sorbitol, les cétones telles que l'acétone, les esters tels que l'acétate de butyle ou l'acétate d'éthyle, le toluène, par exemple.

A titre d'agent épaississant, on peut citer les dérivés de cellulose, les dérivés d'acide polyacrylique, les gommes de guar ou de caroube, ainsi que la gomme de xanthane, par exemple.

Parmi les tensio-actifs, on peut citer notamment les tensio-actifs non-ioniques tels que les alkyl(C₈-C₂₄₎polyglycosides où le nombre de motif glucoside est compris entre 1 et 15 et les tensio-actifs non-ioniques du type polyglycérolé.

Les alkylpolyglycosides sont notamment les produits vendus sous la dénomination APG, tels que les produits APG 300, APG 350, APG 500, APG 550, APG 625, APG base 10-12; les produits vendus par la Société SEPPIC sous les dénominations TRITON CG 110 et TRITON CG 312.

Les composés polyglycérolés sont les dérivés qui résultent de la condensation de 1 à 10, de préférence de 2 à 6 moles de glycidol par mole d'alcool ou d'alphadiol en C₁₀-C₁₄, de diglycolamide d'acides gras en C₁₂-C₁₈, tels que décrits dans les brevets FR 1 477 048, 2 328 763, 2 091 516, 2 169 787.

Les dispersions vésiculaires de lipides amphiphiles ioniques ou non-ioniques citées ci-dessus, peuvent être préparées selon des procédés usuels dont on trouve une liste non limitative dans "Les liposomes en biologie cellulaire et pharmacologie" Edition INSERM/John Libbery Eurotext, (1987), p. 6 à 18.

Pour les compositions sous forme de pâte dentifrice, on peut utiliser les adjuvants et additifs usuels comme des agents de polissage tels que la silice, des agents actifs comme les fluorures tels que le fluorure de sodium, et éventuellement des agents édulcorants tels que le saccharinate de sodium.

Les exemples suivants sont destinés à illustrer l'invention et ne sauraient être considérés comme limitatifs de sa portée.

### EXEMPLES DE PREPARATION

### EXEMPLE I

### Préparation du 1-(2'-F-octyl-éthylthio)-3-(2"-décyl-tétradécyloxy)-2-propanol

Dans un réacteur dc 1 litre, on pèse, sous courant d'azote, 40 g de solution méthanolique de méthylate de sodium (environ 30% - 5,65 meq g⁻¹), puis on ajoute 130 ml de méthanol absolu.

La température est amenée à 2°C, puis 96 g de 2-F-octyl-éthanethiol (0,2 mole) sont ajoutés en 30 minutes en maintenant la température à 2°C.

A la même température, on additionne, en 20 minutes, 89,45 g de 1-(2'-décyl-tétradécyloxy)-3-chloro-2-propanol.

Le bain de glace est retiré. Lorsque le mélange est revenu à la température de 20°C, l'ensemble est porté au reflux du méthanol pendant 3 heures.

Après retour à 25°C, le mélange est filtré sur verre fritté n°4, puis est transvasé dans une ampoule à décanter où l'on récupère la phase inférieure qui se trouve être le 1-(2'-F-octyl-éthylthio)-3-(2"-décyltétradécyloxy)-2-propanol (150 g - 80%).

Le produit peut être purifié sur colonne de silice (éluant heptane/acétate d'éthyle).

On obtient une huile légèrement ambrée.

| Analyse élémentaire : | | | | |
|---|---|---|---|---|
| | % C | % H | % S | % F |
| Calculé | 49,88 | 6,68 | 3,6 | 36,25 |
| Mesuré | 50,07 | 6,72 | 3,28 | 36,06 |

### EXEMPLE II

### Préparation du 1-(2"-F-octyl-éthylthiol-3-(2"-dodécyl-hexadécyloxy)-2-propanol

Dans un réacteur de 1 litre, on pèse sous courant d'azote 20 g de solution méthanolique de méthylate de sodium (environ 30% - 5,65 meq g⁻¹), puis on ajoute 130 ml de méthanol absolu.

La température est amenée à 2°C, puis 48 g de 2-F-octyl-éthanethiol (0,1 mole) sont ajoutés en 20 minutes en maintenant la température à 2°C.

A la même température, on additionne en 15 minutes 50,25 g de 1-(2'-dodécyl-hexadécyloxy)-3-chloro-2-propanol.

Le bain de glace est retiré. Lorsque le mélange est revenu à la température de 20°C, l'ensemble est porté au reflux du méthanol pendant 3 heures.

Après retour à 25°C, le mélange est filtré sur verre fritté n°4 puis est transvasé dans une ampoule à décanter où l'on récupère la phase inférieure qui se trouve être le 1-(2'-F-octyl-éthylthio)-3-(2"-dodécylhexadécyloxy)-2-propanol (66 g - 70%).

Le produit peut être purifié sur colonne de silice (éluant heptane/acétate d'éthyle).

On obtient une cire beige dont le point de fusion est de 30°C.

| Analyse élémentaire : | | | | |
|---|---|---|---|---|
| | % C | % H | % S | % F |
| Calculé | 52,00 | 7,17 | 3,39 | 34,1 |
| Mesuré | 59,84 | 7,06 | 3,66 | 33,9 |

### EXEMPLES DE FORMULATION

### EXEMPLE 1

### CREME DE SOIN - Emulsion huile-dans-eau

On prépare une crème de soin sous forme d'émulsion huile-dans-eau, ayant la composition suivante :

### Phase (A)

- Tensio-actif non-ionique de type hydroxypropyléther, obtenu par condensation, en catalyse alcaline, de 3,5 moles de glycidol sur un mélange d'alphadiols en C₁₁-C₁₄ selon le brevet français FR-2.091.516 2,4 g
- Parahydroxybenzoate de méthyle 0,2 g
- Glycérine 5 g
- Eau 22,06 g

### Phase (A')

- Gel de polyacrylate de glycéryle dans l'eau (50/50), vendu sous la dénomination "HISPAGEL 100" par la Société HISPANO CHEMICA 56,14 g

### Phase (B)

- Alcool cétylique 1 g
- Composé de l'exemple I 5 g
- Huiles amandes d'abricots 5 g
- Huile de sésame 1,5 g
- Triglycéride d'acide caprylique/caprique, vendu sous la dénomination "MIGLYOL 812" par la Société HULS 1,5 g
- Parahydroxybenzoate de propyle 0,2 g

### Mode opératoire :

On chauffe la phase (A) à 80°C. On ajoute, sous agitation, la phase (A'), puis la phase (B) préalablement chauffée à 80°C. On laisse refroidir à température ambiante tout en maintenant l'agitation.

### EXEMPLE 2

### CREME DE SOIN - Emulsion huile-dans-eau

On prépare une crème de soin sous forme d'émulsion huile-dans-eau, ayant la composition suivante :

### Phase (A)

- Tensio-actif non-ionique de type hydroxypropyléther, obtenu par condensation, en catalyse alcaline, de 3,5 moles de glycidol sur un mélange d'alphadiols en C₁₁-C₁₄ selon le brevet français FR-2.091.516 2,4 g
- Parahydroxybenzoate de méthyle 0,2 g
- Glycérine 5 g
- Eau 22,06 g

### Phase (A')

- Gel de polyacrylate de glycéryle dans l'eau (50/50), vendu sous la dénomination "HISPAGEL 100" par la Société HISPANO CHEMICA 56,14 g

### Phase (B)

- Alcool cétylique 1 g
- Composé de l'exemple II 5 g
- Huiles amandes d'abricots 5 g
- Huile de sésame 1,5 g
- Triglycéride d'acide caprylique/caprique, vendu sous la dénomination "MIGLYOL 812" par la Société HULS 1,5 g
- Parahydroxybenzoate de propyle 0,2 g

### Mode opératoire :

On chauffe la phase (A) à 80°C. On ajoute, sous agitation, la phase (A'), puis la phase (B) préalablement chauffée à 80°C. On laisse refroidir à température ambiante tout en maintenant l'agitation.

### EXEMPLE 3

### ROUGE A LEVRES

On prépare un bâton de rouge à lèvres ayant la composition suivante :
- Composé de l'exemple I 1 g
- Ozokérite 14,90 g
- Cire microcristalline 4,90 g
- Cire de Candellila 7,40 g
- Huile de Jojoba 6,20 g
- Huile de ricin 1,20 g
- Lanoline liquide 18,60 g
- Lanoline acétylée 9,90 g
- Huile de vaseline 11,10 g
- Talc 3,70 g
- Micatitane 8,70 g
- D & C Red n°7 Ca lake 5,20 g
- D & C Red n°7 Ba lake 2,80 g
- FD & C Yellow n°5 1 g
- Dioxyde de titane 3,10 g
- Butylhydroxytoluène 0,30 g
- Parfum qs
en mélangeant les huiles à une température de 50 à 60°C. Les pigments et laques organiques sont broyés dans la phase huileuse.

On ajoute alors les cires fondues, le talc et le micatitane, puis le parfum.

La composition est alors coulée dans un moule.

L'application du rouge à lèvres est aisée (glisse facilement) et celui-ci confère de la douceur aux lèvres.

Le composé de l'exemple I peut être remplacé par le composé de l'exemple II.

### EXEMPLE 4

### SHAMPOOING

- Lauryléthersulfate de monoéthanolamine (C₁₂/C₁₄ 70/30) oxyéthyléné à 2,2 moles d'oxyde d'éthylène, vendu sous la dénomination "EMPICOL EMB/FL" par la Société MARCHON 9,8 g MA
- Cocoylbétaine en solution aqueuse à 32% 1,9 g MA
- Hydroxyéthylcellulose réticulée à l'épichlorhydrine quatemisée par la triméthylamide, vendu sous la dénomination "CELQUAT SC 240" par la Société NATIONAL STARCH 0,5 g
- Monoisopropanolamine d'acide de coprah 3 g
- Distéarate de glycol (C₁₆/C₁₈ 30/70) 2 g
- Composé de l'exemple I 0,1 g
- Conservateurs, parfums
- Eau qsp qsq 100g

Le pH est ajusté à 5 par l'acide chlorhydrique.

### EXEMPLE 5

### GEL DE COIFFAGE

- Emulsion huile-dans-eau de copolymère réticulé d'acrylamide et d'acide 2-acrylamido 2-méthylpropane sulfonique de sodium, vendu sous la dénomination "SEPIGEL 305" par la Société SEPPIC 2 g
- Copolymère vinylpyrrolidone/acétate de vinyle (65/35), vendu sous la dénomination "LUVISCOL VA 64" par la Société BASF 1 g
- Composé de l'exemple I 0,2 g
- Eau qsp qsq 100 g

### EXEMPLE 6

### COMPOSITION DE COLORATION

- Alcool oléique polyglycérolé à 2 moles de glycérol 4 g
- Alcool oléique polyglycérolé à 4 moles de glycérol (78% de MA) 5,69 g MA
- Acide oléique 3 g
- Amine oléique oxyéthylénée à 2 moles d'oxyde d'éthylène, vendue sous la dénomination "ETHOMEEN O12" par la Société AKZO 7 g
- Laurylamino succinamate de diéthylaminopropyle, sel de sodium à 55% de MA 3 g MA
- Alcool oléique 5 g
- Diéthanolamide d'acide oléique 12 g
- Propylèneglycol 3,5 g
- Alcool éthylique 7 g
- Dipropylèneglycol 0,5 g
- Monométhyléther de propylèneglycol 9 g
- Métabisulfite de sodium en soluton aqueuse à 35% de MA 0,455 g MA
- Acétate d'ammonium 0,8 g
- Antioxydant, séquestrant qs
- Parfum, conservateur qs
- Ammoniaque à 20% 10 g
- Paraphénylènediamine 0,18 g
- Méthyl-2 N-β-hydroxyéthylamino-5 phénol 0,03 g
- Para-aminophénol 0,06 g
- Résorcinol 0,22 g
- Méta-aminophénol 0,03 g
- N-(β-hydroxyéthyl)amino-5 méthyl-4 amino-2 nitro-1 benzène 0,01 g
- Composé de l'exemple I 1 g
- Eau déminéralisée qsp 100 g

### Mode opératoire :

La composition obtenue est mélangée, poids pour poids, avec de l'eau oxygénée titrant 20 volumes.

Le mélange est appliqué sur des cheveux naturels gris à 90% de blancs.

Les cheveux sont ensuite rincés, lavés, rincés à nouveau, puis séchés. Les cheveux sont teints d'une nuance blond naturel cendré.

### EXEMPLE 7

On prépare une pâte dentifrice en mélangeant les composés suivants :
- Composé de l'exemple I 0,75 g
- Silice précipitée vendue sous la dénomination "SIDENT 22 S" par la Société DEGUSSA 8 g
- Silice précipitée vendue sous la dénomination "SIDENT 9" par la Société DEGUSSA 12 g
- Carboxyméthylcellulose de sodium, vendu sous la dénomination "BLANOSE 12 M 31 XP" par la Société HERCULES 0,8 g
- Sorbitol à 70% dans l'eau 44,8 g MA
- Laurylsulfate de sodium vendu sous la dénomination "EMPICOL LZV/E" par la Société MARCHON à 93% de MA 1,1 g MA
- Fluorure de sodium 0,11 g MA
- Monofluorophosphate de sodium 0,76 g
- Parahydroxybenzoate de méthyle 0,25 g
- Polyéthylèneglycol à 400 moles d'oxyde d'éthylène 2 g
- Saccharinate de sodium 0,15 g
- Arôme, conservateur, colorant qs
- Eau qsp 100 g

### EXEMPLE 8

### CREME DEODORANTE

- Composé de l'exemple I 1 g
- Alcool cétylstéarylique 2 g
- Alcool cétylstéarylique oxyéthyléné à 33 moles d'oxyde d'éthylène 0,5 g
- Trichloro-2,4,4'-hydroxy-2'-diphényléther, vendu sous la dénomination "IRGASAN DP 300" par la Société CIBA GEIGY 0,1 g
- Parahydroxybenzoate de méthyle 0,2 g
- Parahydroxybenzoate de propyle 0,1 g
- Myristate d'isopropyle 12 g
- Eau qsp 100 g

### EXEMPLE 9

### COMPOSITION DE PERMANENTE

On prépare une composition réductrice de déformation permanente des cheveux en procédant au mélange des ingrédients suivants :
- Acide thioglycolique 9 g
- Alcool oléique oxyéthyléné à 20 moles d'oxyde d'éthylène, vendu sous la dénomination "BRIJ 98" par la Société ICI 2 g
- Composé de l'exemple I 0,2 g
- Ammoniaque qs pH=8,2
- Eau déminéralisée qsp 100 g

Cette composition est appliquée sur des cheveux mouillés préalablement enroulés sur des rouleaux. Après avoir laissé agir la composition pendant 15 minutes, on rince abondamment à l'eau, puis on applique la composition oxydante suivante :
- Eau oxygénée qs 8 volumes
- Alcool oléique oxyéthyléné à 20 moles d'oxyde d'éthylène, vendu sous la dénomination "BRIJ 98" par la Société ICI 0,5 g
- Composé de l'exemple I 0,1 g
- Acide citrique qs pH=3,0
- Eau déminéralisée qsp 100 g

On laisse agir la composition pendant environ 5 minutes, puis on enlève les rouleaux, et on rince abondamment la chevelure à l'eau.

Après séchage sous casque, les cheveux présentent de belles boucles.

## Revendications

1. Composés de formule :
R_{F} - (CH₂)ₙ-X-[C₃H₅(OH)]-(Y)_{X}-R_{H} (I)
dans laquelle C₃H₅(OH) représente les structures : 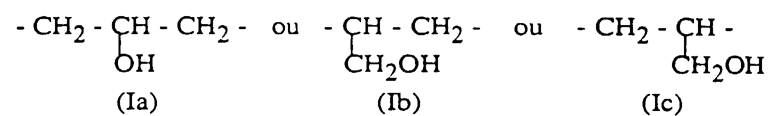
R_{F} représente un radical alkyle perfluoré, linéaire ou ramifié en C₄-C₂₀ ou un mélange de radicaux alkyle perfluorés en C₄-C₂₀;
R_{H} représente un radical alkyle linéaire ou ramifié en C₂₃-C₃₆ ou un mélange de radicaux alkyle linéaires ou ramifiés en C₂₃-C₃₆;
n est compris entre 0 et 4;
X représente O, S, 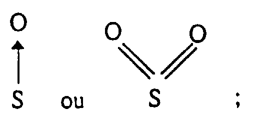
représente O ou 1;
Y représente O, S,

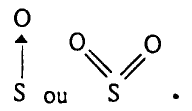

2. Composés selon la revendication 1, caractérisés en ce que :
R_{F} représente un radical alkyle perfluoré en C₆-C₁₀;
R_{H} représente un radical alkyle linéaire ou ramifié en C₂₃-C_{30;}
n est 2;
x est 1;
X représente S, et
Y représente O.

3. Utilisation des composés de formule (I) selon la revendication 1 ou 2, dans des compositions cosmétiques.

4. Composition cosmétique comportant au moins un composé de formule (I) :
R_{F}-(CH₂)ₙ-X-[C₃H₅(OH)]-(Y)_{X}-R_{H} (I)
dans laquelle C₃H₅(OH) représente les structures : 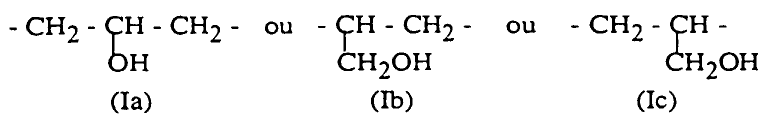
R_{F} représente un radical alkyle perfluoré, linéaire ou ramifié en C₄-C₂₀ ou un mélange de radicaux alkyle perfluorés en C₄-C₂₀;
R_{H} représente un radical alkyle linéaire ou ramifié en C₂₃-C₃₆ ou un mélange de radicaux alkyle linéaires ou ramifiés en C₂₃-C₃₆;
n est compris entre 0 et 4;
X représente O, S, 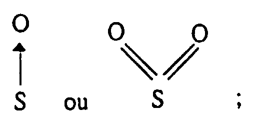
x représente O ou 1;
Y représente O, S,

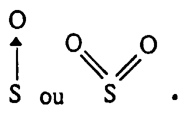

5. Composition cosmétique selon la revendication 4, caractérisée en ce qu'elle est sous forme de lait, crème, lotion huileuse ou oléoalcoolique, gel gras ou oléoalcoolique, dispersion vésiculaire à base de lipides amphiphiles ioniques ou non-ioniques, bâtonnet solide, pâte, spray ou mousse aérosol.

6. Composition cosmétique selon la revendication 4 ou 5, caractérisée en ce qu'elle est sous forme de lait, de crème de soin pour la peau ou les cheveux, de crème, lotion ou lait démaquillant, de crème, gel, lotion ou lait anti-solaire, de crème ou mousse de rasage, de lotion après-rasage, de shampooing ou d'après-shampooing, de déodorant corporel, de pâte dentifrice, de laque, de gel de coiffage, de composition de coloration capillaire, de composition pour la déformation permanente des cheveux, de produit de maquillage des cils, sourcils, ongles, lèvres ou peau, de crème de traitement de l'épiderme, de fond de teint, de bâton de rouge à lèvres, de fard à paupières ou à joues, d'eye-liner, mascara ou de produit de soin pour les lèvres ou les ongles.

7. Composition cosmétique selon l'une des revendications 4 à 6, caractérisée en ce qu'elle comporte 0,1 à 25% en poids du composé de formule (I).

8. Composition cosmétique selon l'une des revendications 4 à 7, caractérisée en ce qu'elle comporte un ou plusieurs adjuvants choisis dans le groupe formé des corps gras, des solvants organiques, des silicones, les épaississants, les adoucissants, des filtres solaires UV-A ou UV-B ou à bande large, des agents anti-mousses, des agents hydratants, des humectants, des parfums, des conservateurs, des tensio-actifs, des charges, des séquestrants, des émulsionnants, des polymères anioniques, cationiques, non-ioniques, amphotères ou leurs mélanges, des anti-perspirants, des agents alcalinisants, des colorants, des pigments, des agents propulseurs, des anti-oxydants et des anti-radicaux libres.

## Patentansprüche

1. Verbindungen der Formel:
R_{F}-(CH₂)ₙ-X-[C₃H₅(OH)]-(Y)_{X}-R_{H} (I)
worin C₃H₅(OH) die Strukturen darstellt: 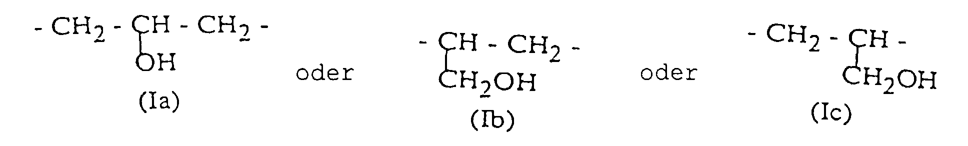
R_{F} einen linearen oder verzweigten perfluorierten C₄₋₂₀-Alkylrest oder eine Mischung aus perfluorierten C₄₋₂₀-Alkylresten und
R_{H} einen linearen oder verzweigten C₂₃₋₃₆-Alkylrest oder eine Mischung aus linearen oder verzweigten C₂₃₋₃₆-Alkylresten und
n 0 bis 4
x O, S, 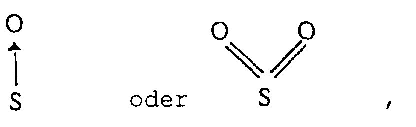
x 0 oder 1 und
Y O, S, 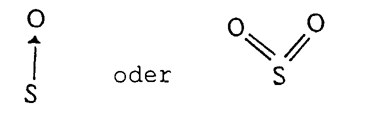 darstellen.

2. Verbindungen gemäß Anspruch 1,
dadurch **gekennzeichnet**, daß
gilt:
R_{F} stellt einen perfluorierten C₆₋₁₀-Alkylrest dar;
R_{H} stellt einen linearen oder verzweigten C₂₃₋₃₀-Alkylrest dar;
n ist 2;
x ist 1;
X stellen S und Y O dar.

3. Verwendung der Verbindungen der Formel (I) gemäß Anspruch 1 oder 2 in kosmetischen Zusammensetzungen.

4. Kosmetische Zusammensetzung, enthaltend mindestens eine Verbindung der Formel (I):
R_{F}-(CH₂)ₙ-X-[C₃H₅(OH)]-(Y)_{X}-R_{H} (I)
worin C₃H₅(OH) die Strukturen darstellt: 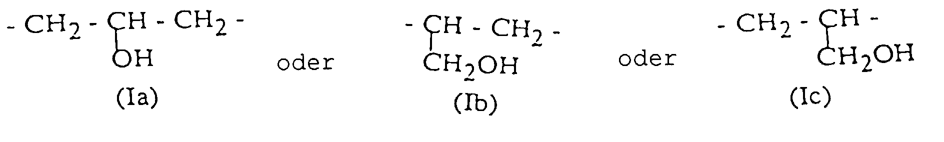
und worin R_{F} einen linearen oder verzweigten perfluorierten C₄₋₂₀-Alkylrest oder eine Mischung aus linearen oder verzweigten perfluorierten C₄₋₂₀-Alkylresten und
R_{H} einen linearen oder verzweigten C₂₃₋₃₆-Alkylrest oder eine Mischung aus linearen oder verzweigten C₂₃₋₃₆-Alkylresten und
n 0 bis 4
x O, S, 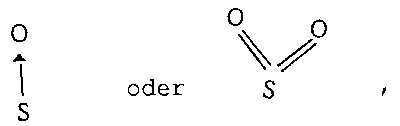
x 0 oder 1 und
Y O, S 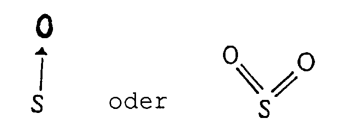 darstellen.

5. Kosmetische Zusammensetzung gemäß Anspruch 4,
dadurch **gekennzeichnet**, daß
sie in Form einer Milch, Creme, ölartigen oder oleoalkoholischen Lotion, eines fettartigen oder oleoalkoholischen Gels, einer bläschenartigen Dispersion auf Basis ionischer oder nicht-ionischer amphiphiler Lipide, eines Feststoffstäbchens, einer Paste, eines Spray oder Aerosol-Schaums vorliegt.

6. Kosmetische Zusammensetzung gemäß Anspruch 4 oder 5,
dadurch **gekennzeichnet**, daß
sie in Form einer Milch oder Creme zur Pflege der Haut oder der Haare, einer Creme, Lotion oder Milch zum Entschminken, einer Creme, eines Gels, einer Lotion oder Milch zum Schutz vor der Sonne, einer Creme oder eines Schaums zur Rasur, einer Lotion zur Anwendung nach einer Rasur, eines Shampoo oder Nach-Shampoo, eines Körperdeodoriermittels, einer Zahpaste, eines Lacks oder Gels für die Frisur, einer Zusammensetzung zur Haarfärbung, einer Zusammensetzung zur Dauerwellung der Haare, eines Produkts zum Schminken der Wimpern, Augenbrauen, Nägel, Lippen oder der Haut, einer Creme zur Behandlung der Epidermis, einer Teint-Grundlage, eines Lippenstifts, einer Schminke für die Augenlider oder Wangen, eines Augen-Liniermittels, einer Wimperntusche oder eines Produkts zur Pflege der Lippen oder Nägel.

7. Kosmetische Zusammensetzung gemäß einem der Ansprüche 4 bis 6,
dadurch **gekennzeichnet**, daß
sie 0,1 bis 25 Gew.% der Verbindung der Formel (I) enthält.

8. Kosmetische Zusammensetzung gemäß einem der Ansprüche 4 bis 7,
dadurch **gekennzeichnet**, daß
sie einen oder mehrere Hilfsstoffe enthält, ausgewählt aus der Gruppe aus Fettkörpern, organischen Lösungsmitteln, Siliconen, Verdickungsmitteln, Weichmachern, Sonnenfilterstoffen für UV-A oder UV-B oder für den langwelligen Bereich, aus Antischaummitteln, Hydratisiermitteln, Feuchtigkeitsmitteln, Parfüm-Produkten, Konservierungsstoffen, oberflächenaktiven Mitteln, Beaufschlagungsmitteln, Sequestriermitteln, Emulgatoren, anionischen, kationischen, nicht-ionischen und amphoteren Polymeren oder aus deren Mischungen, aus Antischweißmitteln, alkalisch stellenden Mitteln, Farbstoffen bzw. Färbemitteln, Pigmenten, Treibmitteln, Antioxidantien und aus Abfangmitteln für freie Radikale.

## Claims

1. A compound with formula:
R_{F}-(CH₂)ₙ-X-[C₃H₅(OH)]-(Y)ₓ-R_{H} (I)
where
C₃H₅(OH) represents the structures: 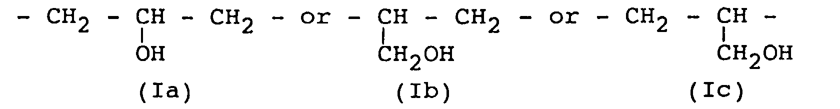
R_{F} represents a perfluorinated, linear or branched C₄-C₂₀ alkyl radical or a mixture of perfluorinated, C₄-C₂₀ alkyl radicals;
R_{H} represents a linear or branched C₂₃-C₃₆ alkyl radical or a mixture of linear or branched C₂₃-C₃₆ alkyl radicals;
n is between 0 and 4;
X represents O, S, 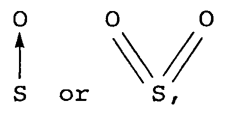
x is 0 or 1;
Y represents O, S,

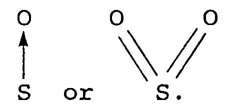

2. A compound according to claim 1 characterized in that:
R_{F} represents a perfluorinated C₆-C₁₀ alkyl radical;
R_{H} represents a linear or branched C₂₃-C₃₀ alkyl radical,
n equals 2;
x equals 1;
X represents S, and
Y represents O.

3. Use of a compound with formula (I) in accordance with claim 1 or claim 2 in a cosmetic composition.

4. A cosmetic composition containing at least one compound with formula (I):
R_{F}-(CH₂)ₙ-X-[C₃H₅(OH)]-(Y)ₓ-R_{H} (I)
where
C₃H₅(OH) represents the structures: 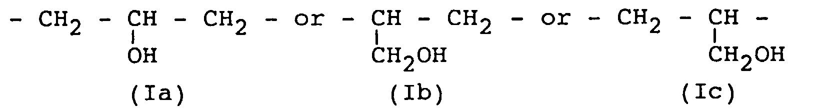
R_{F} represents a perfluorinated, linear or branched C₄-C₂₀ alkyl radical or a mixture of perfluorinated, C₄-C₂₀ alkyl radicals;
R_{H} represents a linear or branched C₂₃-C₃₆ alkyl radical or a mixture of linear or branched C₂₃-C₃₆ alkyl radicals;
n is between 0 and 4;
X represents O, S, 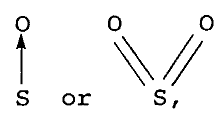
x is 0 or 1;
Y represents O, S,

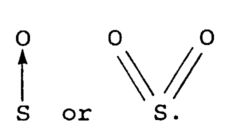

5. A cosmetic composition according to claim 4 characterized in that it is in the form of a milk or cream, oily or oleoalcoholic lotion, oily or oleoalcoholic gel, ionic or non-ionic amphiphilic lipid based vesicular dispersion, solid stick, paste, spray or aerosol foam.

6. A cosmetic composition according to claim 4 or claim 5 characterized in that it is in the form of a milk or cream for skin or hair, a make-up removing cream, lotion or milk, a sun protection cream, gel, milk or lotion, a shaving cream or foam, an aftershave lotion, a shampoo or conditioner, a body deodorant, a toothpaste, a lacquer, a styling gel, a direct hair dye composition, a hair perming composition, a make-up for eyelashes, eyebrows, nails, lips or skin, an epidermal treatment cream, a foundation, lipstick, eye-shadow, blusher, eyeliner, mascara, a lip care product or a nail care product.

7. A cosmetic composition according to any one of claims 4 to 6 characterized in that it contains 0.1% to 25% by weight of the compound with formula (I).

8. A cosmetic composition according to any one of claims 4 to 7 characterized in that it contains one or more additives selected from the group formed by fatty substances, organic solvents, silicones, thickeners, softeners, UV-A or UV-B or broad spectrum solar filters, anti-foaming agents, moisturising agents, humectants, fragrances, preservatives, surfactants, fillers, sequestrating agents, emulsifiers, anionic, cationic, non-ionic or amphoteric polymers and their mixtures, antiperspirants, alkalinizing agents, dyes, pigments, propellants, anti-oxidizing agents and free radical absorbers.
